(19) Europäisches Patentamt
European Patent Office
Office européen des brevets

(11) Publication number: 0 235 726 B1

(12) # EUROPEAN PATENT SPECIFICATION

(45) Date of publication of patent specification: 19.05.93 (51) Int. Cl.5: C12Q 1/68, G01N 33/532

(21) Application number: 87102577.1

(22) Date of filing: 24.02.87

(54) **Rapid detection of nucleic acid sequences in a sample by labeling the sample.**

(30) Priority: 05.03.86 US 836378
29.12.86 US 943006

(43) Date of publication of application:
09.09.87 Bulletin 87/37

(45) Publication of the grant of the patent:
19.05.93 Bulletin 93/20

(84) Designated Contracting States:
AT BE CH DE ES FR GB GR IT LI NL SE

(56) References cited:
EP-A- 0 130 515
EP-A- 0 131 830
EP-A- 0 133 473
EP-A- 0 237 833
WO-A-87/02064
GB-A- 2 095 833
US-A- 4 358 535
US-A- 4 395 486

BIOCHIMICA ET BIOPHYSICA ACTA, vol. 565, no. 1, 1979, Elsevier Science Publishers, Amsterdam (NL); J.P.DAUGHERTY et al., pp. 13-21*

JOURNAL OF BIOLOGICAL CHEMISTRY, vol. 257, no. 15, August 1982, Washington, DC (US); B.A.WHITE et al., pp. 8569-8572*

(73) Proprietor: MILES INC.
One Mellon Center 500 Grant Str.
Pittsburgh, PA 15219-2502(US)

(72) Inventor: Dattagupta, Nanibhushan
470 Prospect Street
New Haven, CT 06511(US)
Inventor: Rae, Peter M.M.
71 Ingram Street
Hamden, CT 06517(US)
Inventor: Rabin, Daniel U.,
24, Stone Street,
Branford, CT 06405(US)
Inventor: Huguenel, Edward D.,
11, Beech Road,
Guilford, CT 06437(US)

(74) Representative: Dänner, Klaus, Dr. et al
c/o Bayer AG Konzernverwaltung RP Patentabteilung
W-5090 Leverkusen 1 Bayerwerk (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

### BACKGROUND OF THE INVENTION

### Field of the Invention

The present application relates to the detection and identification of microorganisms and the detection and identification of particular prokaryotic or eukaryotic DNA sources in a nucleic acid containing test sample.

Still further, the present invention relates to a method for the lysis of whole cells.

### Background Information

#### A. The Detection of Microorganisms

The identification of species of microorganisms in a sample containing a mixture of microorganisms, by immobilizing the DNA from the sample and subjecting it to hybridization with a labelled specimen of species - specific DNA from a known microorganism and observing whether hybridization occurs between the immobilized DNA and the labelled specimen, has been disclosed in PCT patent application No. PCT/US83/01029.

The most efficient and sensitive method of detection of nucleic acids such as DNA after hybridization requires radioactively labeled DNA. The use of autoradiography and enzymes prolongs the assay time and requires experienced technicians.

U.S.P. 4,358,535 to Falkow et al describe infectious disease diagnosis using labeled nucleotide probes complementary to nucleic acid coding for a characteristic pathogen product.

#### B. The Detection of Specific Eukaryotic Sequences

The identification of specific sequence alteration in an eukaryotic nucleic acid sample by immobilizing the DNA from the sample and subjecting it to hybridization with a labeled oligonucleotide and observing whether hybridization occurs between the immobilized DNA and the labeled probe, has been described in EP- patent application No. 86 117 978 filed December 23, 1986; prior art in accordance with Article 54-(3)EPC.

It is known that the expression of a specific gene determines the physical condition of a human being. For example, a change in the beta-globin gene coding sequence from GAG to GTG at the sixth amino acid position produces sickle-beta-globin and a homozygote can have a disease known as sickle cell anemia. Similarly deletion of particular sequences from alpha-globin or beta-globin genes can cause thalassemias. A recent survey, The New Genetics and Clinical Practice, D.J. Weatherall, The Nuffield Provincial Hospitals Trust, (1982), chapter 2 describes the frequency and clinical spectrum of genetic diseases.

Problems associated with genetic defects can be diagnosed by nucleic acid sequence information. The easiest way to detect such sequence information is to use the method of hybridization with a specific probe of a known sequence.

U.S.P. 4,395,486 to Wilson et al describe a method for the direct analysis of sickle cell anemia using a restriction endonuclease assay.

Edward M. Rubin and Yuet Wai Kan, "A Simple Sensitive Prenatal Test for Hydrops Fetalis Caused By $\alpha$-Thalassaemia", The Lancet, January 12, 1985, pp. 75-77 describes a dot blot analysis to differentiate between the genotypes of homozygous alpha-thalassemia and those of the haemoglobin-H disease and alpha-thalassemia trait.

The most efficient and sensitive method of detection of nucleic acids, such as DNA, after hybridization requires radioactively labelled DNA. The use of autoradiography and enzymes prolongs the assay time and requires experienced technicians.

Recently, a non-radioactive method of labelling DNA was described by Ward et al, European Patent Application 63,879. Ward et al, use the method of nick translation to introduce biotinylated U (uracil) residues into DNA, replacing T (thymine). The biotin residue is then assayed with antibiotin antibody or an avidin-containing system. The detection in this case is quicker than autoradiography, but the nick translation method requires highly skilled personnel. Moreover, biotinylation using biotinylated UTP (uridine triphosphate) works only for thymine-containing polynucleotides. The use of other nucleoside

triphosphates is very difficult because the chemical derivatization of A (adenine) or G (guanine) or C (cytosine) (containing $-NH_2$) with biotin requires the skills of trained organic chemists.

C. Cell Lysis

The present invention also provides a method for the efficient lysis of whole cells such that their DNA is released and made available for photochemical labeling. While eukaryotic cells derived from multicellular animals are easily lysed under relatively mild conditions, single cell eukaryotes and prokaryotes, especially Gram positive prokaryotes, are more difficult to lyse due to the complicated chemical nature and extent of cross-linking of their cell walls. Methods do exist for efficiently lysing these refractory organisms, either by chemical-enzymatic or physical means, but these methods are often complicated, time-consuming and inappropriate for preserving the integrity of DNA.

SUMMARY OF THE INVENTION

It is accordingly an object of the present invention to provide a method for detection of microorganisms in a nucleic acid-containing test sample.

It is another object of the invention to provide a method for a simultaneous assay for the presence of more than one nucleic acid sequence.

Another object is to provide a method to identify particular prokaryotic or eukaryotic DNA sequences and a method for distinguishing alleles of individual genes.

Another object of the invention is to provide a simple photochemical method of labeling the unknown test sample.

A still further object of the invention is to use whole chromosomal nucleic acid as the probe and/or as the test sample.

Also the invention relates to the use of oligonucleotides as immobilized probes.

These and other objects and advantages are realized in accordance with the present invention for a method of detecting nucleic acid sequences from microorganisms or eucaryotic sources in a nucleic acid-containing test sample.

The method involves the following:

(a) preparing a test sample comprising labeling the nucleic acids in the test sample and, where the nucleic acids are not in single-stranded form, denaturing such nucleic acids to form single-stranded nucleic acids,

(b) contacting the resulting labeled nucleic acids, under hybridization conditions, with one of more immobilized oligonucleotide or nucleic acid probes hybridizable with the sequence or sequences to be detected, to thereby form hybridized labeled nucleic acids, and

(c) assaying for the hybridized nucleic acids by detecting the label.

According to the invention, a labeled nucleic acid test sample is contacted simultaneously with several different types of DNA probes for hybridization. The nucleic acid test sample is labeled and hybridized with several unlabeled immobilized probes. The positions of the probes are fixed, and the labeled sample detected after hybridization will indicate that the test sample carries a nucleic acid sequence complementary to the corresponding probe.

Nucleic acid probes for several microbiological systems or for different alleles of one or more genes can be immobilized separately on a solid support, for example, nitrocellulose paper. The test sample nucleic acids are labeled and remain in solution. The solid material containing the immobilized probe is brought in contact with the labeled test nucleic acid solution under hybridization conditions. The solid material is washed free of unhybridized nucleic acid and the label is assayed. The presence of the label with one or more of the probes indicates that the test sample contains nucleic acids substantially complementary to those probes and hence originate, for example, from an infection by particular microbiological systems.

Labeling can be accomplished in a whole living cell or a cell lysate, and can be non-isotopic. The nucleic acid can be used for hybridization without further purification.

The present invention also concerns specific lysis conditions to release nucleic acids from both gram positive and gram negative bacteria.

Reagents for releasing and denaturing DNA include sodium hydroxide and lysing agents such as detergents and lysozymes.

Typical hybridization reagents includes a mixture of sodium chloride, sodium citrate, SDS (sodium dodecyl sulfate), bovine serum albumin, nonfat milk or dextran sulfate and optionally formamide.

BRIEF DESCRIPTION OF THE FIGURES

Fig. 1 is an autoradiograph of results of immobilization of an oligonucleotide sequence specific for hemoglobin mutation.

Fig. 2 is a photograph of results of hybridization with labeled genomic DNA for non radioactive detection.

DETAILED DESCRIPTION OF THE INVENTION

The nucleic acid is preferably labeled by means of photochemistry, employing a photoreactive DNA-binding furocoumarin or a phenanthridine compound to link the nucleic acid to a label which can be "read" or assayed in conventional manner, including fluorescence detection. The end product is thus a labeled nucleic acid comprising (a) a nucleic acid component, (b) an intercalator or other DNA-binding ligand photochemically linked to the nucleic acid component, and (c) a label chemically linked to (b).

The photochemical method provides more favorable reaction conditions than the usual chemical coupling method for biochemically sensitive substances. The intercalator and label can first be coupled and then photoreacted with the nucleic acid, or the nucleic acid can first be photoreacted with the intercalator and then coupled to the label.

A general scheme for coupling a nucleic acid, exemplified by double-stranded DNA, to apply a label, is as follows:

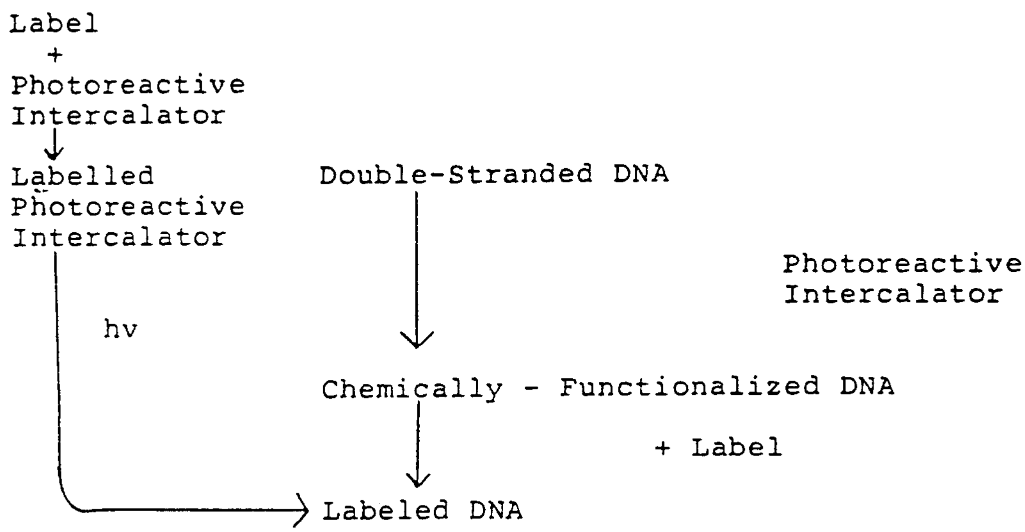


Where the hybridizable portion of the nucleic acid is in a double stranded form, such portion is then denatured to yield a hybridizable single stranded portion. Alternatively, where the labeled DNA comprises the hybridizable portion already in single stranded form, such denaturation can be avoided if desired.

To produce specific and efficient photochemical products, it is desirable that the nucleic acid component and the photoreactive intercalator compound be allowed to react in the dark in a specific manner.

For coupling to DNA, aminomethyl psoralen, aminomethyl angelicin and amino alkyl ethidium or methidium azides are particularly useful compounds. They bind to double-stranded DNA and only the complex produces photoadduct. In the case where labeled double-stranded DNA must be denatured in order to yield a hybridizable single stranded region, conditions are employed so that simultaneous interaction of two strands of DNA with a single photoadduct is prevented. It is necessary that the frequency of modification along a hybridizable single stranded portion of the nucleic acid not be so great as to substantially prevent hybridization, and accordingly there preferably will be not more than one site of modification per 25, more usually 50, and preferably 100, nucleotide bases. Angelicin derivatives are superior to psoralen compounds for monoadduct formation. If a single-stranded DNA nucleic acid is covalently attached to some extra double-stranded DNA, use of phenanthridium and psoralen compounds is desirable since these compounds interact specifically to double-stranded DNA in the dark. The chemistry for the synthesis of the coupled reagents to modify nucleic acids for labeling, described more fully hereinbelow, is similar for all cases.

The nucleic acid component can be single or double stranded DNA or RNA or fragments thereof such as are produced by restriction enzymes or even relatively short oligomers.

The DNA-binding ligands of the present invention used to link the nucleic acid component to the label can be any suitable photoreactive form of known DNA-binding ligands. Particularly preferred DNA-binding ligands are intercalator compounds such as the furocoumarins, e.g., angelicin (isopsoralen) or psoralen or derivatives thereof which photochemically will react with nucleic acids, e.g., 4′-aminomethyl-4,5′-dimethyl angelicin, 4′-aminomethyl-trioxsalen (4′aminomethyl-4,5′,8-trimethyl-psoralen), 3-carboxy-5- or -8-amino-or-hydroxy-psoralen, as well as mono- or bis-azido aminoalkyl methidium or ethidium compounds.

Particularly useful photoreactive forms of intercalating agents are the azidointercalators. Their reactive nitrenes are readily generated at long wavelength ultraviolet or visible light and the nitrenes of arylazides prefer insertion reactions over their rearrangement products (see White et al, Methods in Enzymol., 46, 644 (1977)). Representative intercalating agents include azidoacridine, ethidium monoazide, ethidium diazide, ethidium dimer azide (Mitchell et al, JACS, 104, 4265 (1982)), 4-azido-7-chloroquinoline, and 2-azidofluorene. A specific nucleic acid binding azido compound has been described by Forster et al, Nucleic Acid Res., 13, (1985), 745. The structure of such compound is as follows:

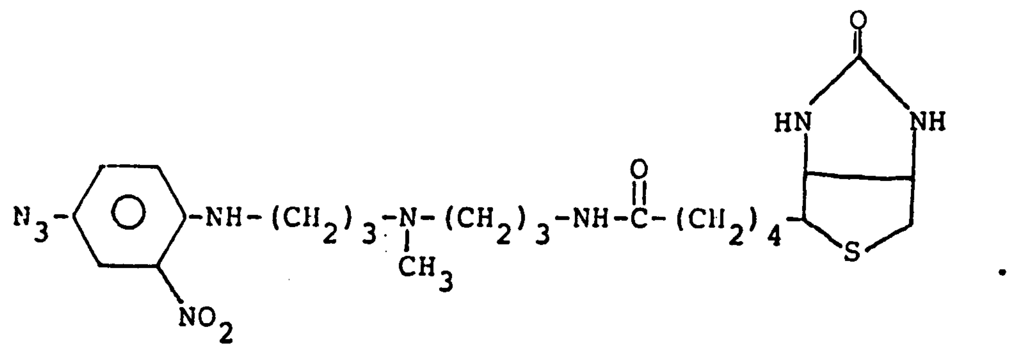

Other useful photoreactable intercalators are the furocoumarins which form (2+2) cycloadducts with pyrimidine residues. Alkylating agents can also be used such as bis-chloroethylamines and epoxides or aziridines, e.g., aflatoxins, polycyclic hydrocarbon epoxides, mitomycin and norphillin A.

Nonlimiting examples of intercalator compounds for use in the present invention include acridine dyes, phenanthridines, phenazines, furocoumarins, phenothiazines and quinolines.

The label which is linked to the nucleic acid component according to the present invention can be any chemical group or residue having a detectable physical or chemical property, i.e., labeling can be conducted by chemical reaction or physical adsorption. The label will bear a functional chemical group to enable it to be chemically linked to the intercalator compound. Such labeling materials have been well developed in the field of immunoassays and in general most any label useful in such methods can be applied to the present invention. Particularly useful are enzymatically active groups, such as enzymes (see Clin. Chem., (1976), 22, 1243), enzyme substrates (see British Pat. Spec. 1,548,741), coenzymes (see U.S. Patent Nos. 4,230,797 and 4,238,565) and enzyme inhibitors (see U.S. Patent No. 4,134,792; fluorescers (see Clin. Chem., (1979), 25, 353), and chromophores including phycobiliproteins; luminescers such as chemiluminescers and bioluminescers (see Clin. Chem., (1979), 25, 512, and ibid, 1531); specifically bindable ligands, i.e., protein binding ligands; and residues comprising radioisotopes such as $^{3}H$, $^{35}S$, $^{32}P$, $^{125}I$, and $^{14}C$. Such labels are detected on the basis of their own physical properties (e.g., fluorescers, chromophores and radioisotopes) or their reactive or binding properties (e.g., enzymes, substrates, coenzymes and inhibitors). For example, a cofactor-labeled nucleic acid can be detected by adding the enzyme for which the label is a cofactor and a substrate for the enzyme. A hapten or ligand (e.g., biotin) labeled nucleic acid can be detected by adding an antibody or an antibody pigment to the hapten or a protein (e.g., avidin) which binds the ligand, tagged with a detectable molecule. An antigen can also be used as a label. Such detectable molecule can be some molecule with a measurable physical property (e.g., fluorescence or absorbance) or a participant in an enzyme reaction (e.g., see above list). For example, one can use an enzyme which acts upon a substrate to generate a product with measurable physical property. Examples of the latter include, but are not limited to, beta-galactosidase, alkaline phosphatase, papain and peroxidase. For in situ hybridization studies, ideally the final product is water insoluble. Other labels, e.g., dyes, will be evident to one having ordinary skill in the art.

The label will be linked to the intercalator compound, e.g., acridine dyes, phenanthridines, phenazines, furocoumarins, phenothiazines and quinolines, by direct chemical linkage such as involving covalent bonds, or by indirect linkage such as by the incorporation of the label in a microcapsule or liposome which in turn is linked to the intercalator compound. Methods by which the label is linked to the intercalator compounds are essentially known in the art and any convenient method can be used to perform the present invention.

Advantageously, the intercalator compound is first combined with label chemically and thereafter combined with the nucleic acid component. For example, since biotin carries a carboxyl group, it can be combined with a furocoumarin by way of amide or ester formation without interfering with the photochemical reactivity of the furocoumarin or the biological activity of the biotin, e.g.,

(i)

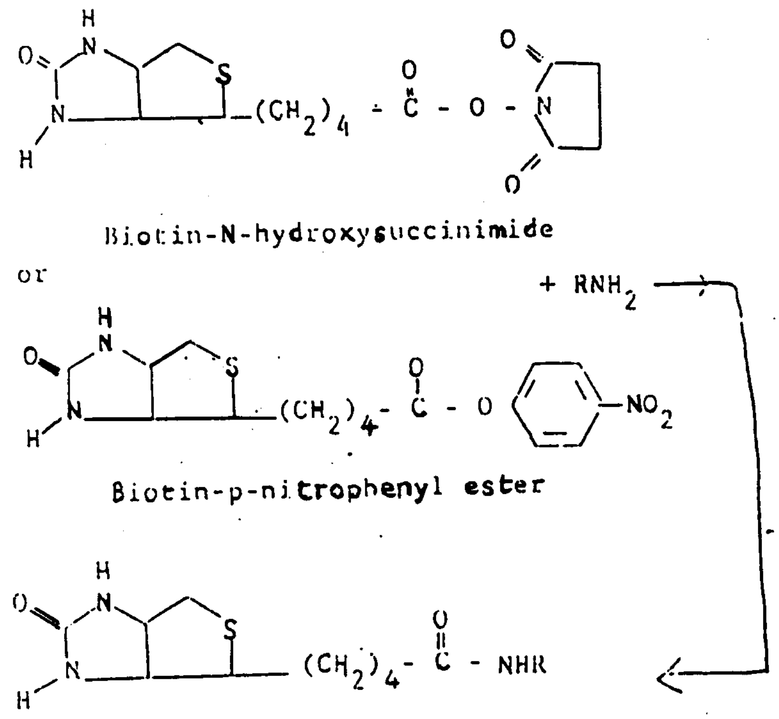

Biotin-N-hydroxysuccinimide

or

+ $RNH_2$

(ii)

Biotin-p-nitrophenyl ester

(iii)

or

carbodiimide

Biotin + ROH → Biotin CO OR

By way of example,

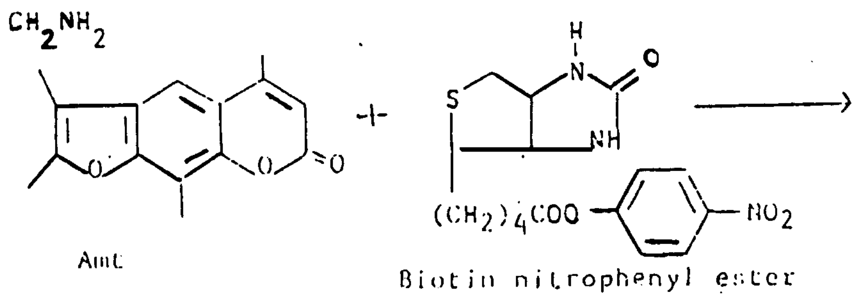

Amt

Biotin nitrophenyl ester

($RNH_2$)

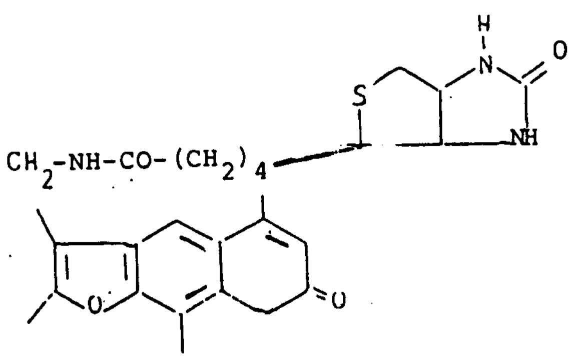

Other aminomethylangelicin, psoralen and phenanthridium derivatives can be similarly reacted, as can phenanthridium halides and derivatives thereof such as aminopropyl methidium chloride, i.e.,

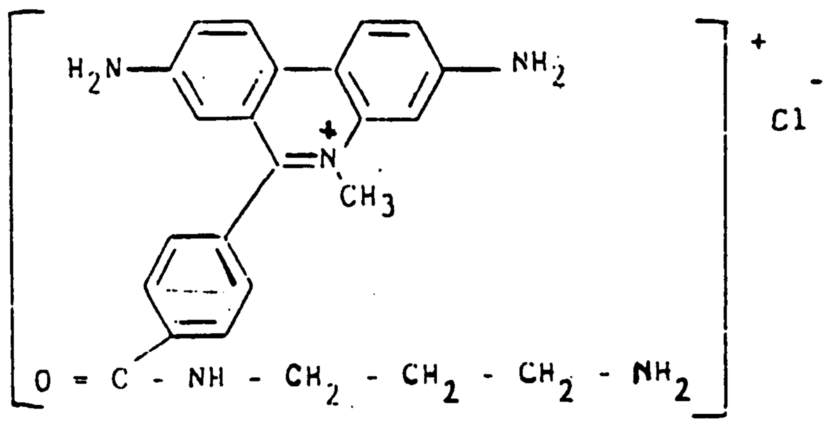

(see Hertzberg et al, J. Amer. Chem. Soc., 104, 313 (1982)).

Alternatively, a bifunctional reagent such as dithiobis succinimidyl propionate or 1,4-butanediol diglycidyl ether can be used directly to couple the photochemically reactive molecule with the label where the reactants have alkyl amino residues, again in a known manner with regard to solvents, proportions and reaction conditions. Certain bifunctional reagents, possibly glutraldehyde may not be suitable because, while they couple, they may modify nucleic acid and thus interfere with the assay. Routine precautions can be taken to prevent such difficulties.

The particular sequence used in making the labeled nucleic acid can be varied. Thus, for example, an amino-substituted psoralen can first be photochemically coupled with a nucleic acid, the product having pendant amino groups by which it can be coupled to the label, i.e., labeling can be carried out by photochemically reacting a DNA binding ligand with the nucleic acid in the test sample. Alternatively, the psoralen can first be coupled to a label such as an enzyme and then to the nucleic acid.

As described in EP- patent application No. 85 116 199.2, filed December 18, 1985, (prior art in accordance with Article 54(3)EPC), the present invention also encompasses a labeled nucleic acid comprising (a) a nucleic acid component, (b) a nucleic acid-binding ligand photochemically linked to the nucleic acid component, (c) a label and (d) a spacer chemically linking (b) and (c).

Advantageously, the spacer includes a chain of up to about 40 atoms, preferably about 2 to 20 atoms, selected from the group consisting of carbon, oxygen, nitrogen and sulfur.

Such spacer may be the polyfunctional radical of a member selected from the group consisting of peptide, hydrocarbon, polyalcohol, polyether, polyamine, polyimine and carbohydrate, e.g., -glycyl-glycyl-glcyl- or other oligopeptide, carbonyl dipeptides, and omega-amino-alkene-carbonyl radical such as $-NH(CH_2)_5-CO-$, a spermine or spermidine radical, an alpha, omega-alkanediamine radical such as $-NH-(CH_2)_6-NH$ or $-HN-CH_2-CH_2-NH$, or the like. Sugar, polyethylene oxide radicals, glyceryl, pentaerythritol, and like radicals can also serve as the spacers.

These spacers can be directly linked to the nucleic acid-binding ligand and/or the label or the linkages may include a divalent radical of a coupler such as dithiobis succinimidyl propionate, 1,4-butanediol diglycidyl ether, a diisocyanate, carbodiimide, glyoxal, glutaraldehyde, or the like.

The spacer can be incorporated at any stage of the process of making the labeled nucleic acid

a-b-d-c

defined hereinabove. Thus, the sequence can be any of the following:

a+b+d+c,

b+d+c+a,

d+c+b+a,

b+d+a+c, etc.

The conditions for the individual steps are well known in chemistry.

If the label is an enzyme, for example, the product will ultimately be placed on a suitable medium and the extent of catalysis will be determined. Thus, if the enzyme is a phosphatase, the medium could contain nitrophenyl phosphate and one would monitor the amount of nitrophenol generated by observing the color. If the enzyme is a beta-galactosidase, the medium can contain o-nitro- phenyl-D-galacto-pyranoside which also will liberate nitrophenol.

The labeled nucleic acid of the present invention is applicable to all conventional hybridization assay formats, and in general to any format that is possible based on formation of a hybridization product or aggregate comprising the labelled nucleic acid. In particular, the unique labelled nucleic acid of the present

invention can be used in solution and solid-phase hybridization formats.

The nucleic acid probe will comprise at least one single-stranded base sequence substantially complementary to or homologous with the sequence to be detected. However, such base sequence need not be a single continuous polynucleotide segment, but can be comprised of two or more individual segments interrupted by nonhomologous sequences. These nonhomologous sequences can be linear or they can be self-complementary and form hairpin loops. In addition, the homologous region of the probe can be flanked at the 3' - and 5' termini by nonhomologous sequences, such as those comprising the DNA or RNA or a vector into which the homologous sequence had been inserted for propagation. In either instance, the probe as presented as an analytical reagent will exhibit detectable hybridization at one or more points with sample nucleic acids of interest. Linear or circular single-stranded polynucleotides can be used as the probe element, with major or minor portions being duplexed with a complementary polynucleotide strand or strands, provided that the critical homologous segment or segments are in single-stranded form and available for hybridization with sample DNA or RNA. Useful probes include linear or circular probes wherein the homologous probe sequence is in essentially only single-stranded form (see particularly, Hu and Messing, Gene, 17:271 (1982)).

The nucleic acid probe of the present invention can be used in any conventional hybridization technique. As improvements are made and conceptually new formats are developed, such can be readily applied to the present probes. Conventional hybridization formats which are particularly useful include those wherein the sample nucleic acid is immobilized on a solid support (solid-phase hybridization) through the probe and those wherein the polynucleotide species are all in solution (solution hybridization).

In solid-phase hybridization formats, the nucleic acid probe is fixed in an appropriate manner in its single-stranded form to a solid support. Useful solid supports are well known in the art and include those which bind nucleic acids either covalently or noncovalently. Noncovalent supports which are generally understood to involve hydrophobic bonding include naturally occurring and synthetic polymeric materials, such as nitrocellulose, derivatized nylon and fluorinated polyhydrocarbons, in a variety of forms such as filters, beads or solid sheets. Covalent binding supports (in the form of filters, beads or solid sheets, just to mention a few) are also useful and comprise materials having chemically reactive groups or groups, such as dichlorotriazine, diazobenzyloxymethyl, and the like, which can be activated for binding to polynucleotides.

It is well known that noncovalent immobilization of an oligonucleotide is ineffective on a solid support, for example, on nitrocellulose paper. The present invention also describes novel methods of oligonucleotide immobilization. This is achieved by phosphorylation of an oligonucleotide by a polynucleotide kinase or by ligation of the 5'-phosphorylated oligonucleotide to produce multi-oligonucleotide molecules capable of immobilization. The conditions for kinase and ligation reaction have been described in standard text books, e.g., Molecular Cloning, T. Maniatis, E.F. Fritsch and J. Sambrook, Cold Spring Harbor Laboratory, (1982), pages 1-123.

For the present invention, the immobile phase of the hybridization system can be a series or matrix of spots of known kinds and/or dilutions of denatured DNA. This is most simply prepared by pipetting appropriate small volumes of native DNA onto a dry nitrocellulose or nylon sheet, floating the sheet on a sodium hydroxide solution to denature the DNA, rinsing the sheet in a neutralizing solution, then baking the sheet to fix the DNA. Before DNA:DNA hybridization, the sheet is usually treated with a solution that inhibits non-specific binding of added DNA during hybridization.

This invention involves the labeling of whole genomic DNA, whole nucleic acids present in cells, whole cell lysate, or unlysed whole cells. Once the labeled material is prepared, it can be used for the detection, i.e., the presence or absence of certain specific genomic sequences by specific nucleic acid hybridization assays.

One method according to the invention involves the separation of cells from a human sample or the human sample directly treated by mixing with a photochemically reactive nucleic acid binding intercalating ligand. The mixture is incubated depending on the type of the sample. If the sample is lysed cells or nucleic acids, it is incubated for a period between a few seconds to five minutes and when whole cells or partially lysed cells are used, incubation between two minutes to two hours is employed. After the mixing and incubation, the whole sample mixture is irradiated at a particular wavelength for the covalent interaction between the photochemically reactive DNA binding ligand and the test sample. Then this labeled material is hybridized under specific hybridization conditions with a specific probe.

After the hybridization, the unreacted unhybridized labeled test sample is removed by washing. After the washing, the hybrid is detected through the label carried by the test sample, which is specifically hybridized with a specific probe.

The present invention is surprising since in a human genomic sample the amount of a single copy gene is very low, for example, if a restriction fragment of one thousand base pair is the region of hybridization,

the probability of such sequence in the whole human genomic sample is one in a million. This conclusion has been derived by assuming from the literature that a human genomic sample has 3 x $10^9$ base pairs and 1000 base pairs will be 1/3,000,000 of that number. Automatically, in a sample of human DNA containing approximately five to ten micrograms of nucleic acids, only 5 to 10 picogram of the corresponding sequences is available and labeling the vast majority of the non-specific DNA should produce more background than the true signal. But after the reaction, it is surprising to observe that the results are not only specific, but also of unexpected higher sensitivity.

Without wishing to be bound by any particular theory of operability, the reason for the unexpected sensitivity may be due to the formation of a network of non-specific nucleic acid hybrids bound to the specific hybrid, thus amplifying the amount of the signal. As has been shown in a typical example, a 19 nucleotide long specific sequence containing plasmid is immobilized and hybridized with 5 microgram equivalent of a test sample which is labeled photochemically and one detects very easily the signal resulted from such hybrid. This could not have been accomplished by any other technique because of the problems associated with the labeling method.

The present invention relates to a novel hybridization technique where probes are immobilized and an eukaryotic nucleic acid sample is labeled and hybridized with immobilized unlabeled probe. A surprising characteristic of the invention is the ability to detect simple or multiple copy gene defects by labeling the test sample. Since there is no requirement for an excess of labeled hybridizing sequence, the present method is more specific. In the present invention, simultaneous detection of different gene defects can be easily carried out by immobilizing specific probes.

For example, using the present invention, one can immobilize oligonucleotide probes specific for genetic defects related to sickle cell anemia and probes for alpha-thalassemias on a sheet of nitrocellulose paper, label the test sample and hybridize the labeled test sample with the immobilized probes. It is surprising that partially purified or unpurified nucleic acid samples (cell lysate or whole cell) can be photochemically labeled with sensitive molecules without affecting the specific hybridizability.

The present invention is also directed to detecting eukaroytes (protists) in samples from higher organisms, such as animals or humans.

Eukaroytes include algae, protozoa, fungi and slime molds.

The term "algae" refers in general to chlorophyll-containing protists, descriptions of which are found in G.M. Smith, Cryptogamic Botany, 2nd ed. Vol. 1, Algae and Fungi, McGraw-Hill, (1955).

Eukaryotic sequences according to the present invention includes all disease sequences except for bacteria and viruses. Accordingly, genetic diseases, for example, would also be embraced by the present invention. Non-limiting examples of such genetic diseases are as follows:

| Area Affected | Diseases |
|---|---|
| Metabolism | Acute intermittent porphyria |
| | Variegate porphyria |
| | $alpha_1$-antitrypsin deficiency |
| | Cystic fibrosis |
| | Phenylketonuria |
| | Tay-Sachs disease |
| | Mucopolysaccharidosis I |
| | Mucopolysaccharidosis II |
| | Galactosaemia |
| | Homocystinuria |
| | Cystinuria |
| | Metachromic leucodystrophy |
| Nervous System | Huntington's chorea |
| | Neurofibromatosis |
| | Myotonic dystrophy |
| | Tuberous sclerosis |
| | Neurogenic muscular atrophies |
| Blood | Sickle-cell anaemia |
| | Beta-thalassaemia |
| | Congenital spherocytosis |
| | Haemophilia A |
| Bowel | Polyposis coli |
| Kidney | Polycystic disease |

| | |
|---|---|
| Eyes | Dominant blindness |
| | `Retinoblastoma |
| Ears | Dominant early childhood deafness |
| | Dominant otosclerosis |
| Circulation | Monogenic hypercholesterolaemia |
| Blood | Congenital spherocytosis |
| Teeth | Dentinogenisis imperfecta |
| | Amelogenisis imperfecta |
| Skeleton | Diaphysial aclasia |
| | Thanatophoric dwarfism |
| | Osteogenes imperfecta |
| | Marfan syndrome |
| | Achondroplasia |
| | Ehlers-Danlos syndrome |
| | Osteopetrosis tarda |
| | Cleft lip/palate |
| Skin | Ichthyosis |
| Locomotor | Muscular dystrophy |

A nucleic acid probe in accordance with the present invention is a sequence which can determine the sequence of a test sample. The probes are usually DNA, RNA, mixed copolymers of ribo- and deoxyribonucleic acids, oligonucleotides containing ribonucleotides or deoxyribonucleotide residues or their modified forms. The sequence of such a probe should be complementary to the test sequence. The extent of complementary properties will determine the stability of the double helix formed after hybridization. The probe can also have covalently linked non-complementary nucleic acids.

The nucleic acid is preferably labeled by means of photochemistry, employing a photoreactive DNA-binding furocoumarin or a phenanthridine compound to link the nucleic acid to a label which can be "read" or assayed in conventional manner, including fluorescence detection.

One use of the present invention is the identification of bacterial species in biological fluids. In one application, samples of urine from subjects having or suspected of having urinary tract infections can provide material for the preparation of labelled DNA(s) or RNAs, while a solid support strip, e.g., made of nitrocellulose or nylon, can contain individual dots or spots of known amounts of denatured purified DNA from each of the several bacteria likely to be responsible for infection.

A urinary tract infection is almost always due to monoclonal growth of one of the following half dozen kinds of microorganism: Escherichia coli (60-90% of UTI), Proteus spp. (5-20% of UTI), Klebsiella spp (3-10% of UTI), Staphylococcus spp. (4-20% of UTI), Streptococcus spp. (2-5% of UTI). Pseudomonas and some other gram negative rods together account for a low percentage of UTI. A common contaminant of urine samples that is a marker of improper sample collection is Lactobacillus.

The concentration of bacteria in a urine sample that defines an infection is about $10^5$ per milliliter.

The format for an unlabeled probe hybridization system applicable to urinary tract infections is to have a matrix of DNAs from the above list of species, denatured and immobilized on a support such as

nitrocellulose, and in a range of amounts appropriate for concentrations of bacterial DNAs that can be expected in samples of labelled unknown.

Standard hybridization with biotinylated whole genome DNA probes takes place in 5 – 10 ml, at a probe concentration of about 0.1 µg/ml; hybridization of probe to a spot containing about 10 ng denatured DNA is readily detectable. There is about 5 fg of DNA per bacterial cell, so that for a sample to contain 1 µg of labelled DNA, it is necessary to collect 2 x $10^8$ bacteria. If an infection produces urine having approximately $10^5$ bacteria/ml, then bacterial DNA to be labeled from a sample is concentrated from 2000 ml. If more than 10 ng unlabeled probe DNA is immobilized in a dot, for example, 100 ng or 1 µg, or if the hybridization volume is reduced, then the volume of urine required for the preparation of labeled unknown is approximately a few tenths of a ml.

A strip of dots containing immobilized, denatured, unlabelled probe DNAs could have the following configuration:

| | 1 µg | 10 ng | 100 pg |
|---|---|---|---|
| Escherichia | o | o | o |
| Proteus | o | o | o |
| Klebsiella | o | o | o |
| Staphylococcus | o | o | o |
| Streptococcus | o | o | o |
| Pseudomonas | o | o | o |
| Lactobacillus | o | o | o |

This procedure involves the labeling of DNA or RNA in a crude cell lysate. Ideally, preparation of labeled sample DNA or RNA will accommodate the following points:

(1) bacteria will be concentrated from a fluid sample by centrifugation or filtration;

(2) bacteria will be lysed under conditions sufficient to release nucleic acids from the most refractory of the organisms of interest;

(3) the labeling protocol will not require purification of labeled nucleic acids from unincorporated precursors, nor the purification of nucleic acids prior to labeling;

(4) the labeling protocol will be sufficiently specific for DNA and/or RNA that proteins, lipids and polysaccharides in the preparation will not interfere with hybridization nor read – out.

In the present invention, there is provided a method for efficiently and rapidly lysing whole cells, including Gram positive bacteria. The method involves contacting cells, e.g., whole cells, with an alkali, e.g., sodium or potassium hydroxide solution in a concentration of 0.1 to 1.6 Normal.

The important features of the present lysis protocol are its relative simplicity and speed. It employs a common chemical that requires no special storage conditions and it lyses even Gram positive organisms with high efficiency, while preserving the properties of the DNA that are important for subsequent steps in the photochemical labeling process.

For the present invention, the immobile phase of the hybridization system can be a series or matrix of spots of known kinds and/or dilutions of denatured DNA. This is most simply prepared by pipetting appropriate small volumes of native DNA or oligonucleotides onto a dry nitrocellulose or nylon sheet, floating the sheet on a sodium hydroxide solution to denature the DNA, rinsing the sheet in a neutralizing solution, then baking the sheet to fix the DNA. Before DNA:DNA hybridization, the sheet is usually treated with a solution that inhibits non specific binding of added DNA during hybridization.

The invention will be further described in the following non – limiting examples wherein parts are by weight unless otherwise expressed.

## Examples

### Example 1: Preparation of Labelling Compound

The preparation of the labeling compound required 1 – amino – 17 – N – (Biotinylamido) – 3,6,9,12,15 pentaoxaheptadecane. This compound was prepared in the following four steps:

(a) 3,6,9,12,15 pentaoxapheptadecane 1,17−diol ditosylate was synthesized.
(b) 1,17−dipthalimido derivative of 3,6,9,12,15 pentaoxaheptadecane was prepared.
(c) 1,17−diamino derivative of 3,6,9,12,15 pentaoxaheptadecane was prepared.
(d) 1−amino, 17−biotinylamido derivative of 3,6,9,12,15 pentaoxaheptadecane was prepared.

Example 1(a): Preparation of 3,6,9,12,15−Pentaoxaheptadecane−1,17−diol Ditosylate

To a stirred solution containing 50 g of hexaethylene glycol (0.177 mol) and 64 ml of triethylamine (39.36 g, 0.389 mol) in 400 ml of $CH_2Cl_2$ at 0°C was added dropwise a solution containing 73.91 g of p−toluenesulfonyl chloride (0.389 mol) in 400 ml of $CH_2Cl_2$ over a 2.5 hour period. The reaction mixture was then stirred for one hour at 0°C and then heated to ambient temperature for 44 hours. The mixture was then filtered and the filtrate was concentrated in vacuo. The resulting heterogeneous residue was suspended in 500 ml of ethyl acetate and filtered. The filtrate was then concentrated in vacuo to a yellow oil which was triturated eight times with 250 ml portions of warm hexane to remove unreacted p−toluenesulfonyl chloride. The resulting oil was then concentrated under high vacuum to yield 108.12 g of a yellow oil (quantitative yield).

| Analysis: Calculated for $C_{26}H_{38}O_{11}S_2$ | | |
|---|---|---|
| Calc.:<br>found: | C, 52.87;<br>C, 52.56; | H, 6.48.<br>H, 6.39. |

PMR: (60 MHz, $CDCl_3$) δ : 2.45 (s, 6H); 3.4−3.8 (m, 20H); 4.2 (m, 4H); 7.8 (AB quartet, J=8Hz, 8H).
IR: (neat) $cm^{-1}$: 2870, 1610, 1360, 1185, 1105, 1020, 930, 830, 785, 670.

Example 1(b): Preparation of 1,17−Diphthalimido−3,6,9,12,15−pentaoxaheptadecane

A stirred suspension containing 108 g of 3,6,9,12,15−pentaoxaheptadecane−1,17−diol ditosylate (0.183 mol), 74−57 g of potassium phthalimide (0.403 mol), and 700 ml of dimethylacetamide was heated at 160−170°C for 2 hours and was then cooled to room temperature. The precipitate was filtered and washed with water and acetone to yield 53.05 g of product as a white powder which was dried at 55°C (0.1 mm). mp 124−126°C.

A second crop of product was obtained from the dimethylacetamide filtrate by evaporation in vacuo and the resulting precipitate with was successively washed ethyl acetate, water, and acetone. The resulting white powder was dried at 55°C (0.1 mm) to yield an additional 9.7 g of product. mg 124.5−126.5°C. The combined yield of product was 62.82 g (68% yield).

Analysis: (For first crop)

| Calculated for $C_{28}H_{32}N_2O_9.1/2H_2O$ | | | |
|---|---|---|---|
| Calc.:<br>found: | C, 61.19;<br>C, 61.08; | H, 6.05;<br>H. 6.15; | N, 5.09.<br>N, 5.05. |

(For second crop)

| Calculated for $C_{28}H_{32}N_2O_9$ | | | |
|---|---|---|---|
| Calc.:<br>found: | C, 62.21;<br>C, 61.78; | H, 5.97;<br>H, 6.15; | N, 5.18.<br>N, 5.13. |

PMR: (60 MHz, dmso−$d_6$) δ : 3.5 (s, 8H); 3.6 (s, 8H); 3.8 (bt, J=3Hz, 8H): 8.1 (s, 8H).
IR: (KBr) $cm^{-1}$: 2890, 1785, 1730, 1400, 1100, 735.

Example 1(c): Preparation of 1,17 – Diamino – 3,6,9,12,15 – Pentaoxaheptadecane

A solution containing 60 g of 1,17 – diphthalimido – 3,6,9,12,15 – pentaoxaheptadecane (0.118 mol), 14.8 g of hydrazine hydrate (0.296 mol), and 500 ml of ethanol were heated with mechanical stirring in a 100 ° C oil bath for three hours. The mixture was then cooled and filtered. The resultant filter cake was washed four times with 300 ml portions of ethanol. The combined filtrates were concentrated to yield 32.35 g of a yellow apaque glassy oil. The evaporative distillation at 150 – 200 ° C (0.01 mm) gave 22.82 g of a light yellow oil (69% yield). lit. b.p. 175 – 177 ° C (0.07 mm).

PMR: (60 MHz, $CDCl_3$) δ : 1.77 (s, 4H, $NH_2$); 2.85 (t, J = 5Hz, 4H); 3.53 (t, J = 5Hz, 4H); 3.67 (m, 16H).

IR: ($CHCl_3$) $cm^{-1}$: 3640, 3360, 2860, 1640, 1585, 1460, 1350, 1250, 1100, 945, 920, 870.

Mass Spectrum: (EI) m/e = 281.2 (0.1%, M + 1).
(FAB) m/e = 281.2 (100%, M + 1).

| Analysis: For $C_{12}H_{28}N_2O_5$.1/2 $H_2O$ | | | |
|---|---|---|---|
| Calc.: | C, 49.80, | H, 10.10; | N, 9.68. |
| found: | C, 50.36, | H, 9.58; | N, 9.38. |

Literature Reference: W. Kern, S. Iwabachi, H. Sato and V. Bohmer, Makrol. Chem., 180, 2539 (1979).

Example 1(d): Preparation of 1 – Amino – 17 – N – (Biotinylamido) – 3,6,9,12,15 – pentaoxaheptadecane

A solution containing 7.2 g of 1,17 – diamino – 3,6,9,12,15 – pentaoxaheptadecane (25 mmol) in 75 ml of DMF under an argon atmosphere was treated with 3.41 g of N – succinimidyl biotin (10 mmol) added in portions over 1.0 hour. The resulting solution was stirred for four hours at ambient temperature. TLC ($SiO_2$, 70:10.1 $CHCL_3$ – $CH_3OH$ – conc. $NH_4$ OH) visualized by dimethylaminocinnamaldehyde spray reagent showed excellent conversion to a new product (Rf = 0.18). The reaction mixture was divided in half and each half was absorbed onto $SiO_2$ and flash – chromatographed on 500 g of $SiO_2$ – 60 (230 – 400 mesh) using a 70:10.1 $CHCl_3$ – $CH_3OH$ – conc. $NH_4OH$ solvent mixture. Fractions containing the product were polled and concentrated to a yield 2.42 g of a gelatinous, waxy solid. The product was precipitated as a solid from isopropanol – ether, washed with hexane, and dried at 55 ° C (0.1 mm) to give 1.761 g of a white powder (35% yield).

| Analysis: Calculated for $C_{22}H_{42}N_4O_7S$.3/2 $H_2O$: | | | |
|---|---|---|---|
| | C, 49.51; | H, 8.50; | N. 10.49. |
| found: | C, 49.59; | H, 8.13; | N, 10.39. |

PMR: (90 MHz, dmso – $d_6$) δ : 1.1 – 1.7 (m, 6H); 2.05 (t, J = 7Hz, 2H); 2.62 (t, J = 4Hz, 1H); 2.74 (t, J = 4Hz, 1H); 3.0 – 3.4 (m, 14H). 3.50 (s, 14H); 4.14 (m, 1H); 4.30 (m, 1H); 6.35 (d, J = 4Hz, 1H); 7.80 (m, 1H).

CMR: (22.5 MHz, dmso – $d_6$) δ : 25.2, 28.0, 28.2, 35.1, 40.6, 55.3, 59.2, 61.1, 69.6, 69.8, 71,2, 162.7, 172.1.

IR: (KBr) $cm^{-1}$: 2900, 2850, 1690, 1640, 1580, 1540, 1450, 1100.

Mass Spectrum (FAB) m/e: 507.3 (M + 1, 56%)

Example 2: Preparation of 4′ – Biotinyl – PEG – 4,5′ – dimethylangelicin

A solution of 203 mg of 1 – amino – 17 – N – (biotinylamido) – 3,6,9.12,15 – pentaoxaheptadecane (0.4 mmol) in 1 ml of DMF under an argon atmosphere was treated with 78 mg of N,N – carbonyldimidazole (0.48 mmol). The resulting mixture was stirred for four hours and was then treated with 55 mg of 4′ – aminomethyl – 4,5′dimethylingelicin hydrochloride (0.2 mmol), 140 μl of diisopropylethylamine, and 100 μl of DMF. The resulting mixture was stirred overnight at 50 ° C. The mixture was then evaporated onto $SiO_2$ in vacuo and the resultant impregnated solid flash was chromatographed on 60 g of $SiO_2$ (230 – 400 mesh) eluted with 1.5 liters of 7% $CH_3$ – $CHCl_3$ followed by 1 liter of 10% $CH_3OH$ – $CHCl_3$. Fractions containing the

product were pooled and concentrated to yield 72 mg of a glassy solid (47% yield).

PMR: (90 MHz, dmso-$d_6$): $\delta$ 1.1-1.8 (m, 6H); 2.04 (bt, J=7Hz, 2H); 2.5 (s, 6H); 2.56 (m, 1H); 2.74 (bd, J=4Hz, 1H); 2.8-3.4 (m, 14H); 3.40 (m, 14H); 4.14 (m, 1H); 4.25 (m, 1H); 4.40 (bd, J=6Hz, 2H); 6.5 (m, 1H); 6.35 (s, 1H); 7.02 (s, 1H); 7.45 (d, J=8Hz, 1H); 7.62 (d, J=8Hz, 1H); 7.80 (m, 1H).

CMR: (22.5 MHz, dmso-$d_6$) $\delta$ : 11.9, 18.9, 25.3, 28.2 28.3, 33.4, 35.2, 55.4, 59.2, 61.0, 69.2, 69.6, 69.8, 70.0, 89.0, 107.8, 112.0, 113.1, 114.3, 120.6, 121.6, 153.6, 154.4, 155.6, 157.9, 159.5, 162.7, 172.1.

Literature Reference: F. Dall'Acqua, D. Vedaldi, S. Caffieri, A. Guiotto, P. Rodighiero, F. Baccichetti, F. Carlassare and F. Bordin, J. Med. Chem., 24, 178 (1981).

Example 3: Colorimetric or Chemiluminescent Detection of the Nucleic Acid Hybrids

Example 3(a): Colorimetric Detection

Colorimetric detection of the biotinylated hybrids is carried out following the procedure and kit developed by Bethesda Research Laboratories (BRL), Gaithersburg, Maryland 20877, U.S.A. The procedure is described in detail in a manual supplied with a kit by BRL, entitled "Products for Nucleic Acid Detection", "DNA Detection System Instruction Manual", Catalogue No. 8239SA.

Example 3(b): Chemiluminescent Detection

Chemiluminescent detection of the biotinylated hybrids is identical to the above method: the filters with the hybrids are saturated with BSA (bovine serum albumin) by immersing the paper in 3% BSA at 42°C for 20 minutes. Excess BSA is removed by taking the paper out of the container, and blotting it between two pieces of filter paper. The paper is then incubated in a solution containing Streptavidin (0.25 mg/ml, 3.0 ml total volume), for 20 minutes at room temperature. It is then washed three times with a buffer containing 0.1 M Tris-HCl, pH 7.5, 0.1 M NaCl, 2mM $MgCl_2$, 0.05% "TRITON® X-100". Next the filter is incubated with biotinylated horseradish peroxidase (0.10 mg/ml) for 15 minutes at room temperature. This is followed by three washings with 0.1 M Tris-HCl, pH 7.5, 0.1 M NaCl, 2 mM $MgCl_2$ and 0.05% Triton X-100, and one washing with 10 mM Tris (pH 8.0) buffer. Chemiluminescent activation is conducted in two ways. (1) Spots are punched out and the discs containing the DNA are placed in a microtiter plate with wells that are painted black on the sides. After the punched paper circles are placed in the microtiter place wells, 0.8 ml buffer containing 40 mM Tris and 40 mM ammonium acetate (pH 8.1) is added to each well. Then 10 $\mu$l of 1:1 mixture of 39 mM Luminol (in DMF) and 30 mM $H_2O_2$ (in water) is added. Light emission is recorded on a "POLAROID®" instant film by exposing it directly in the film holder. Alternatively (2), the paper is soaked in a solution containing 1:1 mixture of 0.5 mM Luminol and $H_2O_2$ and wrapped with a transparent "SARAN WRAP®". The light emission is recorded on a " POLAROID®" film as above.

Example 4: General Method of Labeling the Test Sample Nucleic Acids

High molecular weight DNA from a patient's sample is isolated by a method described in U.S.P. 4,395,486 (Wilson et al). The nucleic acid is dissolved in 10 mM borate buffer (pH 8.0) to a final concentration of approximately 20 $\mu$g/ml. To the nucleic acid solution "angelicin-peg-biotin" in aqueous solution is added to a final concentration of 10 $\mu$g/ml. The mixture is then irradiated at long wavelength irradiation for about 60 minutes using a black ray UVL 56 lamp. The product is ready for hybridization without purification. However, the product can be purified by dialysis or alcohol precipitation (U.S.P. 4,395,486) as is usually followed for nucleic acids.

Instead of nucleic acids, whole cell lysate can also be labeled following an identical procedure. The lysis is conducted by boiling the cells with 0.1 N sodium hydroxide, followed by neutralization with hydrochloric acid.

When whole cells are used, the mixture of "PEG-ang-bio" and cells are incubated for at least 60 minutes prior to irradiation for efficient transport of the ligands. Many different variations of the above described methods can be adopted for labeling.

Example 5:

Alpha-thalassemia is associated with gene deletion. The detection of gene deletion by hybridization in a dot/slot blot format requires that the total amount of sample and its hybridizability are accurately known. Since the beta-globin gene is a single copy gene, simultaneous hybridization of a sample with beta-globin and alpha-globin and their relative amounts will indicate the amount of alpha-globin with the sample.

The format and hybridization conditions are the same as Rubin and Kan, supra, except probes, not test DNA, is immobilized. Hybridization conditions are also similar. The detection is done by using the BRL kit described supra following BRL's specifications.

The hybridization detection process are conducted in three steps as follows:

Step 1: Immobilization of the Probes

As described in Rubin and Kan, supra, 1.5 kb PstI fragment containing $alpha_2$ globin gene is used as a probe for alpha-thalassemia and for the beta-globin gene a 737 base pair probe produced by the digestion of pBR beta Pst (4.4 kb) is used. The beta-globin gene probe has been described in U.S.P. 4,395,486 (column 4). For the detection of gene deletion related to alpha-thalassemia, the amount of starting nucleic acid, hybridization efficiency and control samples are needed. The present invention avoids these problems by simultaneous hybridization with a single copy essential gene (e.g., beta-globin gene) when similar amounts of probes are immobilized side by side, labeled sample is hybridized, relative strength of signal intensity is a measure of relative amount of gene dosage present in the sample.

The probes (0.5, 1, 3 and 5 µg per 100 µl) are suspended in 10 mM tris HCl (pH 7) buffer, denatured with 20 µl 3 M sodium hydroxide, at 100 °C, for 5 minutes, an equivalent volume of 2 M ammonium acetate, pH 5.0 is added to neutralize the solution, immediately after neutralization the probes for beta- and alpha-globin genes are applied in parallel rows to nitrocellulose filter paper under vacuum in a slot blot manifold, purchased from Scleicher and Schuell, (Keeni, New Hampshire, U.S.A.). The filter is then dried in vacuum at 80 °C for 60 minutes. It is then prehybridized for 4 hours in a mixture containing 50 mM sodium phosphate (pH 7) 45 mM sodium citrate, 450 mM sodium chloride, 50% (v/v) formamide, 0.2% each (w/v) of polyvinyl pyrrolidine, "FICOLL® 400" and bovine serum albumin and 0.2 mg/ml alkali boiled salmon sperm DNA and 0.15 mg/ml yeast RNA.

Step 2: Labeling of the Test Sample

This was described above.

Step 3: Hybridization

The nitrocellulose strip containing the immobilized probes are hybridized with the labeled test sample in plastic bags (e.g., "SEAL-A-MEAL®", "SEAL and SAVE®", etc.). Hybridization solution is the same as prehybridization solution plus 10% dextran sulphate. Hybridization is done at 42 °C for 16 hours. After hybridization detection of biotin is conducted with a kit and procedure supplied by Bethesda Research Laboratory, Maryland, U.S.A., (catalogue No. 8239SA). Results of relative intensity of alpha- and beta-regions are used to estimate the extent of deletion of alpha-globin genes:

No signal on the alpha-globin side: all 4 alpha-globin genes missing.

Signal on the alpha-globin side is half as strong as on the corresponding beta-side: 3 alpha-globin genes missing.

Signals on alpha and beta side equivalent: 2 alpha-globin genes missing.

Signals on alpha side is stronger than the corresponding beta side (2 alpha = 3 beta): 1 alpha-globin gene missing.

Example 6: Immobilization of an Oligonucleotide Sequence Specific for Hemoglobin Mutation

It is known that an oligonucleotide cannot be easily immobilized onto nitrocellulose paper by a simple adsorption process. The present invention encompasses three different methods to incorporate an oligonucleotide sequence into a larger molecule capable of adsorption.

Method 1: Two oligonucleotides, one a 43mer and the other a 16-mer, have been chemically synthesized in an automated synthesizer (Applied Biosystem 380B) by the phosphoramidite-method and

phosphorylated at the 5′ end by a T4 – polynucleotide kinase mediated process according to Maniatis et al, Molecular Cloning, page 122. These oligonucleotides contain a segment of a 19 nucleotide long sequence specific for the detection of the mutation associated with sickle cell anemia.

43mer A & S (A = normal globin gene; S = sickle globin gene) were kinased according to Maniatis et al, Molecular Cloning, page 122, in two separate reactions, namely, one with $^{32}P$ – ATP and one with no radioactive label. 0.4 μg $^{32}P$ – 43mer and 0.6 mg cold 43mer were mixed and purified on a spun column (G – 25med in TE (Tris EDTA buffer)) to a final volume of 40 μl. Two dilutions were spotted on S & S (Schleicher & Schuell) nitrocellulose and nytran (nylon) membranes at 50 and 0.5 ng.

Method 2: The phosphorylated oligonucleotide products of method 1 were further elongated by making multimers of sequences by a ligase mediated process. The principle is described as follows:

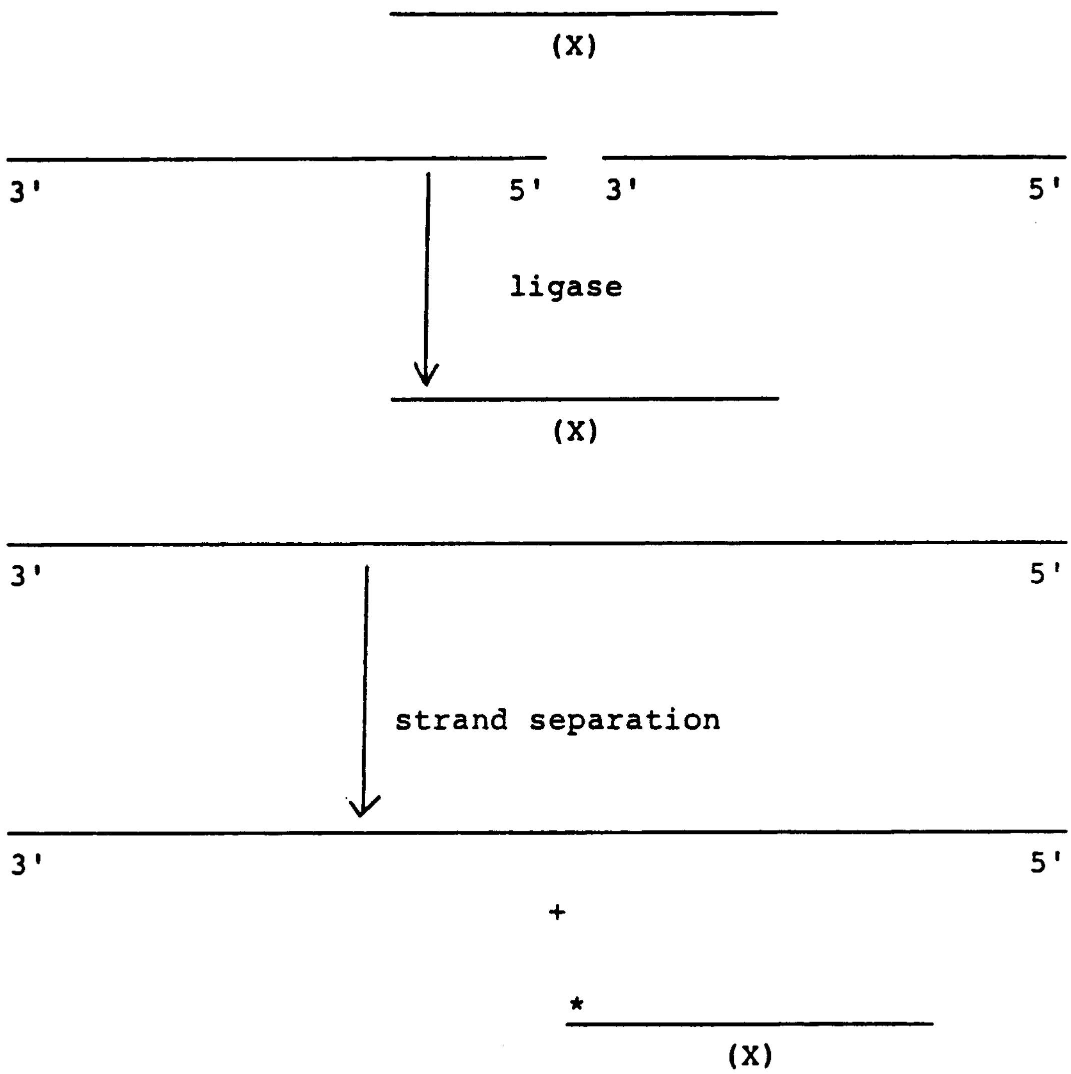


The product being of a higher molecular weight than an oligonucleotide it should be immobilizable by adsorption on to a nitrocellulose paper.

Aqueous solutions containing 4μg of $^{32}P$43mer and 3.7 μg 16mer linker (X) were mixed and dried under vacuum. 6 mg of cold kinased 43mer was added and the sample was heated to 55 ° C and cooled slowly to 0 ° C to anneal. Ligation was carried out in 20 μl total reaction volume with 800 units of ligase (Pharmacia) at 15 ° C for 4 hours. 1 mg (2 μl) was purified on a spun column (G – 25med in TE) to a final volume of 40 μl. Two dilutions were spotted on nitrocellulose nylon membranes at 50 and 0.5 ng.

Method 3: The same as method 2, but ligation was not conducted. Instead of ligation, cross linking was conducted with an intercalator to keep the double stranded regions intact. Hence, the cross linked molecule will have several oligonucleotide sequences covalently linked to each other.

2 µg of $^{32}$P43mer (for sequence P-50) was added to 2.9 mg of a 16mer (for sequence P-50) linker and purified on a spun column (G25med in TE) to a final volume of 40 µl. 6 mg of kinased 43mer was added and the samples were heated to 55°C and cooled slowly to 0°C to anneal. 25 µl of intercalation compound aminomethyltrioxsalen was added and the sample was irradiated for 30 minutes on ice in 500 µl total 10 mM borate buffer pH 8.2 with a long wave UV lamp model (UVL-21, λ = 366 nm).

The probes modified by all three methods were then immobilized on to nitrocellulose and nylon paper and hybridized with labelled oligonucleotides. The results indicate that the sequence are immobilizable and hybridization fidelity remains intact.

Two dilutions of the products of methods 1 to 3 were spotted on nitrocellulose and nylon membranes at 50 and 0.5 ngs.

Whole filters were baked for 30 minutes in 80°C vacuum oven and prehybridized in blotto (5% nonfat dry milk, 6XSSC, 20 mM Na-pyrophosphate) for 30 minutes in 50°C oven.

Hybridization was carried out with primer extended 19′A & 19S′ probes at 50°C for one hour (3 strips/probe).

Filters were stringently washed for 15 minutes at room temperature in 6XSSC with slight agitation and 2 x 10 minutes at 57°C.

Air-dried filters were place on Whatman paper and autoradiographed at -70°C overnight.

The results presented in Fig. 1 surprisingly indicate specific hybridization are obtained by immobilizing oligonucleotide probes.

Example 7: Hybridization with labeled genomic DNA for Non Radioactive Detection

Human normal genomic (XX) DNA was photolabeled with "biotin-PEG-angelicin" (BPA) in 10 mM borate buffer pH 8.2 at a weight ratio of 0.3 to 1 (BPA:DNA) for 15 minutes on ice with a long wave UV lamp model UVL-21, λ = 366 nm. No purification is necessary.

Target DNA oligonucleotides were directly immobilized on S & S nitrocellulose in 1 µl aliquots at the following concentration, and then baked in an 80°C vacuum oven for 30 minutes. The amounts of the different immobilized probes are as follows:

| | | | | |
|---|---|---|---|---|
| 43-mer | (A) | - | Kinased (method 1) | 200 ng |
| 43-mer | (A) | | | 200 ng |
| 43-mer | (S) | - | Kinased (method 1) | 200 ng |
| 43-mer | (S) | | | 200 ng |
| M1319Ass | | | | 50 ng |
| M1319Sss | | | | 50 ng |
| M13737Ass | | | | 50 ng |
| BRL Commercially biotinylated DNA | | | | 200 pg |
| pUC19 | | | | 50 ng |

43-merA: 5′ CTGCT$_4$AATCTTAAGGAT$_3$AAT$_4$CTCCTGȦGGAGAAGTCT
GCT$_4$AATCTTAA5$_1$GGAT$_3$AAT$_4$CT 3′

• =T for 43-mer S

16-mer (Common to both A and S)

3′ - TTAGAATTCCTAAATT-5′

Filters were prehybridized in blotto (5% nonfat dry milk, 6XSSC, 20 mM Na-pyrophosphate) for 30 minutes in a 45° C $H_2O$ bath.

All 4 strips were hybridized in 2 mls solution containing 2 μg labeled XX DNA containing normal beta-globin gene (hybridization solution was blotto with 10% PEG) for 2 hours in 45° C in a $H_2O$ bath.

A stringency wash was carried out as follows:

1 X 20′ at room temperature in 6XSSC

2 X 20′ at temperatures indicated in Fig. 2 with very little agitation.

50 ml centrifuge tubes were used for elevated temperature washes. Results are shown in Fig. 2.

Detection of biotin in the hybrid was carried out according to the Bethesda Research Laboratory, Bethesda, Maryland, U.S.A., manual using their kit for biotin detection. The results indicated specific hybridization.

Example 8: Immobilization of Whole Genomic DNA As Probes

Tens of milligram to gram amounts of DNA were prepared in the following manner from bacterial cells harvested from fermentor cultures. Bacteria were collected by centrifugation from 10 liter nutrient broth cultures grown in a New Brunswick Scientific Microferm Fermentor. Generally, cells in concentrated suspension were lysed by exposure to an ionic detergent such as SDS (Na dodecyl sulfate), then nucleic acids were purified from proteins and lipids by extraction with phenol and/or chloroform (J. Marmur, J. Mol. Biol., 3, 208-218, 1961). RNA was removed from the nucleic acids preparation by treatment of the DNA solution with 0.2 mg/ml ribonuclease at 37° C, then DNA was precipitated from solution by the addition of two volumes of ethanol. Bacterial DNA redissolved from the precipitate in a low salt buffer such as TE (10 mM Tris-HCl, pH 7.5, 1mM $Na_2$ EDTA) was characterized with respect to purity concentration and molecular size, then approximately 1 microgram aliquots were denatured and immobilized as spots on nitrocellulose or nylon membranes for hybridization (Kafatos et al., Nucleic Acids, Res. 7, 1541-1552, (1979)). Denaturation was accomplished by exposure of the DNA with approximately 0.1 N NaOH. After denaturation the solution was neutralized, then the membrane was rinsed in NaCl/Tris-HCl, pH 7.5, and dried.

Example 9: Processing of a Test Sample for Cellular DNA Labeling

Samples of urine, for example (although the following can equally apply to suspensions of material form gonorrhea-suspect swabs, from meningitis-suspect cerebrospinal fluid, from contamination-suspect water samples, etc.), are centrifuged or filtered to wash and concentrate any bacteria in the sample. The bacteria are then lysed by exposure to either (i) 2 mg/ml lysozyme or lysostaphin then exposure to approximately 90° C heat, (ii) 0.1 to 1.6 N NaOH, or (iii) 1% Na dodecyl sulfate. After (ii) NaOH, the cell lysate solution is neutralized before labelling; after (iii) detergent lysis, DNA labelling is preceded by removal of the SDS with 0.5 M K acetate on ice. Angelicin should be able to permeate intact cells so that DNA labeling can be accomplished before cell lysis. This in situ labeling simplifies the extraction procedure, as alkaline or detergent lysates can be incorporated directly into a hybridization solution.

Prior to hybridization, the labeled sample is denatured, and it should also preferably be reduced to short single stranded lengths to facilitate specific annealing with the appropriate unlabeled probe DNA. Methods of denaturation are known in the art. These methods include treatment with sodium hydroxide, organic solvent, heating, acid treatment and combinations thereof. Fragmentation can be accomplished in a controlled way by heating the DNA to approximately 80° C in NaOH for a determined length of time, and this, of course, also denatures the DNA.

Example 10: Labeling of the Products of Example 9

(i) A test sample of about 10ml urine will contain $10^4$ or more infectious agents. After separation by centrifugation and washing, the pretreated cell lysate (step 2) was resuspended in 0.2 ml 10 mM sodium borate buffer (pH approximately 8). To this suspension, 10 μg of photolabelling reagent dissolved in ethanol (10 mg/ml), was added and mixed by shaking on a vortex mixer. The mixture was then irradiated at 365 nm for 30 minutes with a UVGL 25 device at its long wavelength setting. The UVGL device is sold by UVP Inc., 5100 Walnut Grove Avenue, P.O. Box 1501, San Gabriel, CA 91778, U.S.A.

(ii) The sample was also labeled with N-(4-azido-2-nitrophenyl)-N′-(N-d-biotinyl-3-aminopropyl)-N′-methyl-1,3-propanediamine (commercially available from BRESA, G.P.O. Box 498, Adelaide, South Australia 5001, Australia), following the procedure described by Forster et al (1985),

supra for DNA.

(iii) When unlysed cells were used, the cell suspension in 0.2 ml 10 mM borate was incubated with the photoreagent for 1 hour prior to irradiation.

Example 11: Hybridization of the Products of Examples 8 and 10

Prior to hybridization, the membrane with spots of denatured unlabeled probe DNA was treated for up to 2 hours with a "prehybridization" solution to block sites in the membrane itself that could bind the hybridization probe. This and the hybridization solution, which also contained denatured labeled sample DNA, was comprised of approximately 0.9 M $Na^+$, 0.1% SDS, 0.1 – 5% bovine serum albumin or nonfat dry milk, and optionally formamide. With 50% formamide, the prehybridization and hybridization steps were done at approximately 42 °C; without, the temperature was approximately 68 °C. Prehybridized membranes can be stored for some time. DNA hybridization was allowed to occur for about 10 minutes or more, then unbound labeled DNA was washed from the membrane under conditions such as 0.018 M $Na^+$ (0.1 x SSC), 0.1% SDS, 68 °C, that dissociate poorly base paired hybrids. After posthybridization washes, the membrane was rinsed in a low salt solution without detergent in anticipation of hybridization detection procedures.

Example 12: Detection of a Nucleic Acid Hybrid with Immunogold

Affinity isolated goat antibiotin antibody (purchased from Zymed Laboratories, San Francisco, California, U.S.A.) was adsorbed onto colloidal gold (20 nm) following the method described by its supplier (Janssen instruction booklet, Janssen Life Sciences Products, Piscataway, New Jersey, U.S.A.) and reacted with hybridized biotinylated DNA after blocking as in a colorimetric method. The signals were silver enhanced using a Janssen (B2340 BEERSE, Belgium) silver enhancement kit and protocol.

Example 13: Detection of Urinary Tract Infection in a Urine Sample

Urine samples were collected from a hospital where they were analyzed by microbiological methods and the results were kept secret until the hybridization diagnosis was conducted. Then they were compared ascertain the validity of the hybridization results.

1 ml of clinical sample (urine) suspected of UTI infection was centrifuged in a Brinkman micro centrifuge for 5 minutes. Then 0.1 ml of 1.2 N sodium hydroxide was added and the suspension was heated to 100 °C to lyse the cells. The suspension was then diluted to 1 ml with 10 mM sodium borate buffer pH 8 and was neutralized with hydrochlorine acid to a pH of 7. To the solution, 50 μg "biotin – PEG – angelicin" (see Example 2) is added and the mixture was irradiated with a UVL 56 long wavelength UV lamp for 15 minutes. The irradiated sample (0.1 ml) was added to 3 ml 3XSSC of 5% nonfat dry milk 10% PEG with 0.2 M sodium pyrophosphate and hybridization was conducted with probes (whole genomic DNA) immobilized onto nitrocellulose paper at 68 °C for 5 minutes to overnight. After hybridization detection was conducted according to Examples 3 or 12, the spots or the photographs were visually interpreted for the presence of specific bacteria in the test sample. A spot of human DNA was also present in the nitrocellulose paper for the detection of leucocytes. The presence of leucocytes was further verified with a common method using "LEUKOSTIX®" (Miles Laboratories, Elkhart, Indiana, U.S.A.).

Typical results (Tables 1 and 2) indicate that the hybridization diagnosis produces similar results in a shorter time then the corresponding microbiological assays. The present invention not only provides information related to species identification, but also the leucocyte content in a clinical sample.

TABLE 1

DIAGNOSIS OF CLINICAL URINE SAMPLES

| * HOSPITAL DIAGNOSIS | APPLICANTS' HYBRIDIZATION RESULTS | DETECTION SYSTEM |
|---|---|---|
| NEG | NEG | GOLD |
| NEG | NEG | GOLD |
| NEG | NEG | GOLD |
| NEG | E.c.-M | CHEMI |
| NEG | E.c.-VW | GOLD |
| NEG | E.c.-VW | GOLD |
| S+, C- | NEG | GOLD |
| S+, C- | E.c.-S | CHEMI |
| S+, C- | E.c.-S, Kl.-M | CHEMI |
| S+, C- | NEG | GOLD |
| S+, C- | NEG | GOLD |
| S+, C- | NEG | GOLD |
| S+, C- | E.c.-VW | GOLD |
| S+, C- | NEG | GOLD |
| S+, C- | E.c.-VW | GOLD |
| S+, C- | NEG | GOLD |
| S+, C- | NEG | GOLD |
| 100,000/mL E.c. | E.c.-S | GOLD |
| 100,000/mL E.c. | E.c.-S | CHEMI |
| 100,000/mL E.c. | E.c.-W | GOLD |
| 50,000/mL E.c. | E.c.-M | CHEMI |
| 50,000/mL E.c. | NEG | GOLD |
| E. coli | E.c.-S, Kl.-M | CHEMI |
| E. coli | E.c.-VS, Kl.-S | CHEMI |
| E. coli | E.c.-S, Kl.-S | CHEMI |
| E. coli/Klebsiella mix | E.c.-S, Kl.-W | GOLD |
| E. coli/Staph mix | E.c.-S, St.-M | CHEMI |

Table 1 cont'd

| HOSPITAL DIAGNOSIS | APPLICANTS' HYBRIDIZATION RESULTS | DETECTION SYSTEM |
|---|---|---|
| Klebsiella spp. | E.c.-M, Kl.-W | CHEMI |
| 100,000/mL K. pneumoniae | E.c.-W, Kl-VW | GOLD |
| Enterobacter spp. | NEG** | GOLD |
| 100,000 Candida | NEG** | GOLD |
| 100,000/mL Proteus | Pr.-S, E.c.-W | GOLD |
| 10,000/mL Strep | NEG | CHEMI |
| Mixture of 3 unidentified Gm(+) | NEG | GOLD |

* diagnosis conducted by streaking urine on an agar plate and treating the plate under conditions so that the infectious organism can grow.

** Enterobacter/Candida probes not included in the hybridization assay, therefore, negative results are not surprising; given the high stringency conditions employed in the assay, cross-hybridization with species related to Enterobacter was not detected.

Abbreviations: VS=very strong; S=strong; M=medium; W=weak; and VW=very weak hybridization signals; GOLD=detection method according to Example 12; CHEMI=chemiluminescent detection according to Example 3(b)

Applicants' hybridization results represent the results of a subjective interpretation of the intensity of the hybridization signals obtained after detection. DNAs from the organisms listed in column two are the only ones for which any hybridization signal was obtained. The panel of DNAs used for hybridization included E. coli ("E.c."), Klebsiella pneumoniae ("Kl"), Proteus vulgaris ("Pr"), Pseudomonas aeruginosa, Staphylococcus epidermatis ("SE"), Streptococcus faecalis and Homo sapiens.

TABLE 2

COMPARISON OF AMES LEUKOSTIX ASSAY WITH APPLICANT'S ASSAY

| "LEUKOSTIX®" RESULT | APPLICANTS' HYBRIDIZATION RESULT | DETECTION SYSTEM |
|---|---|---|
| 3+ | VS | GOLD |
| 3+ | S | CHEMI |
| 3+ | S | CHEMI |
| 3+ | M | CHEMI |
| 3+ | M | CHEMI |
| 3+ | S | GOLD |
| 3+ | S | GOLD |
| 3+ | VS | GOLD |
| 3+ | VS | GOLD |
| 3+ | VS | GOLD |
| 3+ | VS | GOLD |
| 2+ | S | CHEMI |
| 2+ | S | CHEMI |
| 2+ | S | CHEMI |
| 2+ | S | CHEMI |
| 2+ | S | GOLD |
| 2+ | S | GOLD |
| 2+ | S | GOLD |
| 2+ | S | GOLD |
| 2+ | S | GOLD |
| 1+ | S | GOLD |
| 1+ | VS | GOLD |
| 1+ | VW | GOLD |
| TRACE/1+ | M | CHEMI |
| TRACE | VS | CHEMI |
| TRACE | W | GOLD |
| TRACE | W | GOLD |
| TRACE | VS | GOLD |
| NEG | S | CHEMI |
| NEG | S | CHEMI |
| NEG | M | CHEMI |
| NEG | VW | GOLD |

Table 2 cont'd

| "LEUKOSTIX®" RESULT | APPLICANTS' HYBRIDIZATION RESULT | DETECTION SYSTEM |
|---|---|---|
| NEG | VW | GOLD |
| NEG | NEG | GOLD |
| NEG | NEG | GOLD |
| NEG | W | GOLD |
| NEG | W | GOLD |
| NEG | W | GOLD |

The hybridization results summarized in column 2 of Table 2 represent subjective interpretations of the intensity of hybridization signal obtained when labeled urine samples described in Table 1 were hybridized with genomic human DNA.

The "LEUKOSTIX®" assay is a colorimetric reagent strip assay. Color development on the reagent strip is compared to a chart provided with the assay reagent strips and ranges from negative (no color development) to 3+ (very strong color development).

Example 14: Lysis of Cells

A 1.0 mL aliquot of cell suspension was centrifuged and the cell pellet resuspended in 100 µL of unbuffered NaOH solution. The sample was then exposed to high temperature for a short time and then diluted to the original volume using 10 mM borate buffer. The pH of the solution was then adjusted to neutral with HCl.

Table 4 shows the efficiency of lysis of two different Gram positive cocci, Staphylococcus epidermidis and Streptococcus faecalis, at varying NaOH concentrations at either 68°C or 100°C. In this Example, the absorbance of the 10 mL aliquots at 600 nm was recorded before centrifugation. After centrifugation, the cell pellets were resuspended in varying concentrations of NaOH (100 µL) and duplicate samples of each exposed to 68°C for 10 minutes or 100°C for 5 minutes. Each sample was then diluted to 1.0 mL and the absorbance at 600 nm again recorded. Since the beginning and ending volumes are identical, the beginning and ending absorbance at 600 nm provides a direct measurement of lysis efficiency.

Whereas Gram negative organisms lysed efficiently in as low as 0.1 N NaOH, Table 4 shows clearly that efficient lysis is a function of both NaOH concentration and temperature, such that higher NaOH concentrations are required as the incubation temperature decreases. At 100°C (maximum temperature at 1 atmosphere) a concentration of at least 1.6 N NaOH was required for efficient lysis of S. epidermidis and S. faecalis. If lower temperatures are desirable or necessary, then higher concentrations of NaOH will be required to maintain lysis efficiency.

TABLE 3

EFFICIENCY OF LYSIS OF GRAM POSITIVE BACTERIA AT VARIOUS CONCENTRATIONS OF NaOH AT 68°C and 100°C

Streptococcus faecalis

| | 100°C/5 Minutes | | | 68°C/10 Minutes | | |
|---|---|---|---|---|---|---|
| [NaOH] | OD600 PRE | OD600 POST | %LYSIS | OD600 PRE | OD600 POST | %LYSIS |
| 0 N | 0.475 | 0.366 | 23 | 0.512 | 0.357 | 30 |
| 0.1 | .509 | .261 | 50 | .513 | .238 | 54 |
| 0.2 | .512 | .194 | 62 | .514 | .259 | 50 |
| 0.4 | .504 | .175 | 65 | .513 | .150 | 71 |
| 0.8 | .506 | .113 | 78 | .505 | .147 | 71 |
| 1.2 | .498 | .082 | 84 | .498 | .150 | 70 |
| 1.6 | .487 | .061 | 88 | .426 | .099 | 77 |

Staphylocuccus epidermidis

| | 100°C/5 Minutes | | | 68°C/10 Minutes | | |
|---|---|---|---|---|---|---|
| [NaOH] | OD600 PRE | OD600 POST | %LYSIS | OD600 PRE | OD600 POST | %LYSIS |
| 0 N | 0.667 | 0.558 | 16 | 0.690 | 0.560 | 19 |
| 0.1 | .681 | .396 | 42 | .701 | .441 | 37 |
| 0.2 | .674 | .296 | 60 | .699 | .414 | 41 |
| 0.4 | .699 | .183 | 74 | .730 | .309 | 58 |
| 0.8 | .705 | .091 | 87 | .715 | .187 | 74 |
| 1.2 | .680 | .070 | 90 | .719 | .090 | 88 |
| 1.6 | .693 | .035 | 95 | .660 | .040 | 94 |

## Claims

**1.** A method for detecting one or more polynucleotide sequences from microorganisms or eucaryotic sources in a nucleic-acid containing test sample, comprising the steps of:

(a) preparing a test sample comprising labeling the nucleic acids in the test sample and, where the nucleic acids are not in single-stranded form, denaturing such nucleic acids to form single-stranded nucleic acids,

(b) contacting the resulting labeled nucleic acids, under hybridization conditions, with one of more immobilized oligonucleotide or nucleic acid probes hybridizable with the sequence or sequences to be detected, to thereby form hybridized labeled nucleic acids, and

(c) assaying for the hybridized nucleic acids by detecting the label.

**2.** The method of claim 1 wherein the test sample is a biological fluid and the method is further characterized by the steps of:

(i) centrifuging or filtering a sample of said fluid to isolate cells therefrom,

(ii) treating the cell-containing fraction from step (i) to lyse cells and to release nucleic acids therefrom,

(iii) specifically labeling released sample nucleic acids directly in the lysate produced in step (i) without purification or isolation of specific nucleic acid sequences.

**3.** The method of claim 2 wherein the biological test sample is selected from urine and blood.

**4.** The method of any of claims 1 to 3 wherein said labeling is performed by photochemically reacting a labeled form of a nucleic acid binding ligand with the sample nucleic acids.

**5.** The method of any of claims 1 to 4 wherein said eucaryotic sources are selected from algae, protozoa, fungi, slime molds, and mammalian cells.

6. The method of any of claims 1 to 4 wherein said microorganisms are selected from Escherichia, Proteus, Klebsiella, Staphylococcus, Streptococcus, Pseudomonas and Lactobacillus.

7. The method of claim 2 wherein cells in the sample are lysed by treatment with alkali.

8. The method of any of claims 1 to 7 wherein the label is selected from protein binding ligands, haptens, antigens, fluorescent compounds, dyes, radioactive isotopes, and enzymes.

9. The method of any of claims 1 to 8 wherein the probes are immobilized by covalent coupling or adsorption to a solid surface.

10. The method of claim 9 wherein the probes are immobilized in the form of dots on a solid support strip.

**Patentansprüche**

1. Verfahren zum Nachweis einer oder mehr Polynucleotidsequenzen aus Mikroorganismen oder eukaryontischen Quellen in einer nucleinsäurehaltigen Probe, umfassend die Schritte:
   (a) Herstellen einer Probe, umfassend das Markieren der Nucleinsäuren in der Probe, und, wo die Nucleinsäure nicht in einzelsträngiger Form vorliegen, Denaturieren solcher Nucleinsäuren unter Bildung einzelsträngiger Nucleinsäuren,
   (b) Inkontaktbringen der erhaltenen, markierten Nucleinsäuren unter Hybridisierungsbedingungen mit einer oder mehreren immobilisierten Oligonucleotid- oder Nucleinsäuresonden, die hybridisierbar mit der Sequenz oder den nachzuweisenden Sequenzen sind, unter Bildung von hybridisierten, markierten Nucleinsäuren, und
   (c) Test auf hybridisierte Nucleinsäuren durch Nachweis der Markierung.

2. Verfahren nach Anspruch 1, worin die Sonde eine biologische Flüssigkeit ist, und das Verfahren ferner charakterisiert ist durch die Schritte:
   (i) Zentrifugieren oder Filtrieren einer Probe der Flüssigkeit zur Isolierung der Zellen daraus,
   (ii) Behandlung der zellhaltigen Fraktion aus Schritt (i) unter Lyse der Zellen und Freisetzen der Nucleinsäuren daraus,
   (iii) spezifisches Markieren der freigesetzten Nucleinsäuren direkt im Lysat, hergestellt aus Schritt (i) ohne Reinigung oder Isolierung der spezifischen Nucleinsäuresequenzen.

3. Verfahren nach Anspruch 2, worin die biologische Probe ausgewählt ist aus Urin und Blut.

4. Verfahren nach einem der Ansprüche 1 bis 3, worin die Markierung erfolgt durch fotochemisches Umsetzen einer markierten Form eines nucleinsäurebindenden Liganden mit den Proben-Nucleinsäuren.

5. Verfahren nach einem der Ansprüche 1 bis 4, worin die eukaryontischen Quellen ausgewählt sind aus Algen, Protozon, Pilzen, Schleimpilzen und Säugerzellen.

6. Verfahren nach einem der Ansprüche 1 bis 4, worin die Mikroorganismen ausgewählt sind aus Escherichia, Proteus, Klebsiella, Staphylococcus, Streptococcus, Pseudomonas und Lactobacillus.

7. Verfahren nach Anspruch 2, worin die Zellen in der Probe durch Behandlung mit Alkali lysiert werden.

8. Verfahren nach einem der Ansprüche 1 bis 7, worin die Markierung ausgewählt ist aus proteinbindenden Liganden, Heptenen, Antigenen, Fluoreszenzverbindungen, Farbstoffen, radioaktiven Isotopen und Enzymen.

9. Verfahren nach einem der Ansprüche 1 bis 8, worin die Sonden durch kovalente Kopplung oder Adsorption an eine feste Oberfläche immobilisiert werden.

10. Verfahren nach Anspruch 9, worin die Sonden in Form von Dots auf einem festen Trägerstreifen immobilisiert werden.

## Revendications

**1.** Procédé pour détecter une ou plusieurs séquences polynucléotidiques provenant de micro–organismes ou d'organismes eucaryotiques dans un échantillon d'essai contenant des acides nucléiques, comprenant les étapes consistant :
(a) à préparer un échantillon d'essai par marquage des acides nucléiques dans l'échantillon d'essai et, lorsque les acides nucléiques ne sont pas sous forme monocaténaire, par dénaturation de ces acides nucléiques pour former des acides nucléiques monocaténaires,
(b) à mettre en contact les acides nucléiques marqués résultants, dans des conditions d'hybridation, avec l'une de plusieurs sondes d'oligonucléotides ou d'acides nucléiques immobilisés hybridables avec la ou les séquences à détecter, pour former ainsi des acides nucléiques marqués hybridés, et
(c) à analyser les acides nucléiques hybridés par détection du marqueur.

**2.** Procédé suivant la revendication 1, dans lequel l'échantillon d'essai est un liquide biologique et le procédé est caractérisé en outre par les étapes consistant :
(i) à centrifuger ou filtrer un échantillon dudit liquide pour en isoler les cellules,
(ii) à traiter la fraction contenant les cellules de l'étape (i) pour provoquer la lyse des cellules et pour libérer les acides nucléiques de ces cellules,
(iii) à marquer spécifiquement les acides nucléiques libérés de l'échantillon directement dans le lysat produit dans l'étape (i) sans purification ni séparation de séquences d'acides nucléiques spécifiques.

**3.** Procédé suivant la revendication 2, dans lequel l'échantillon biologique d'essai est choisi entre l'urine et le sang.

**4.** Procédé suivant l'une quelconque des revendications 1 à 3, dans lequel le marquage est effectué par réaction photochimique d'une forme marquée d'un ligand de liaison aux acides nucléiques avec les acides nucléiques d'échantillon.

**5.** Procédé suivant l'une quelconque des revendications 1 à 4, dans lequel les organismes eucaryotiques servant de source sont choisis entre des algues, des protozoaires, des champignons, des myxomycètes et des cellules de mammifères.

**6.** Procédé suivant l'une quelconque des revendications 1 à 4, dans lequel les micro–organismes sont choisis entre Escherichia, Proteus, Klebsiella, Staphylococcus, Streptococcus, Pseudomonas et Lactobacillus.

**7.** Procédé suivant la revendication 2, dans lequel les cellules présentes dans l'échantillon sont lysées par traitement avec une substance alcaline.

**8.** Procédé suivant l'une quelconque des revendications 1 à 7, dans lequel le marqueur est choisi entre des ligands de liaison aux protéines, des haptènes, des antigènes, des composés fluorescents, des colorants, des isotopes radioactifs et des enzymes.

**9.** Procédé suivant l'une quelconque des revendications 1 à 8, dans lequel les sondes sont immobilisées par couplage covalent ou adsorption à une surface solide.

**10.** Procédé suivant la revendication 9, dans lequel les sondes sont immobilisées sous forme de taches sur une bandelette solide de support.

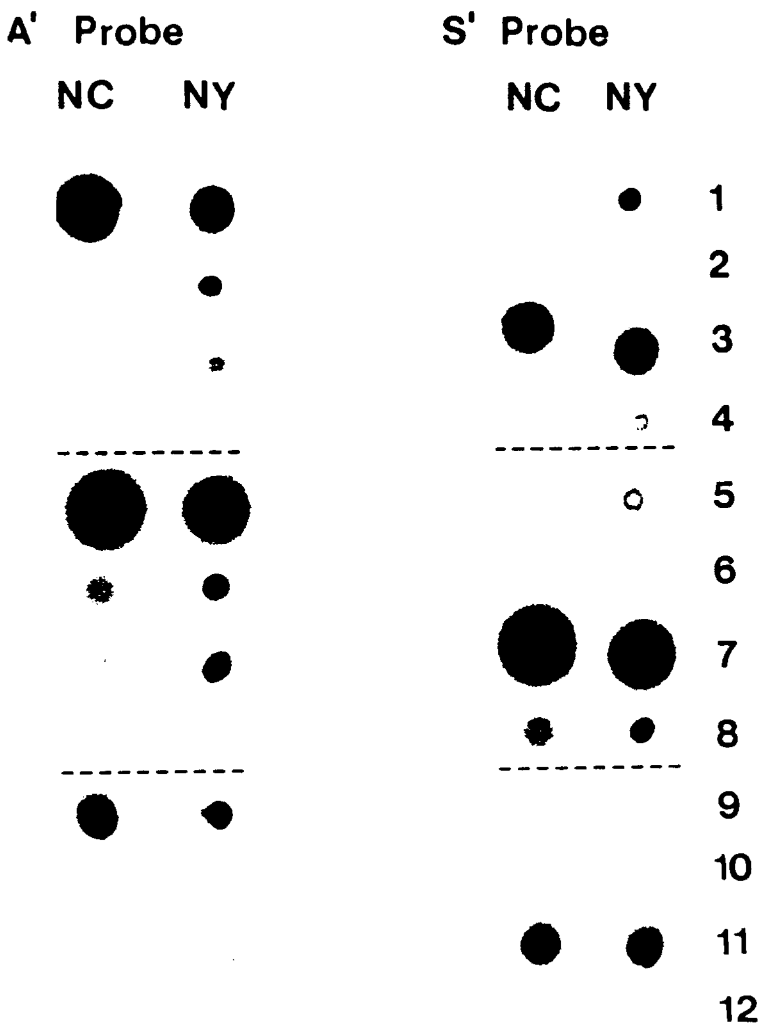


FIG. 1

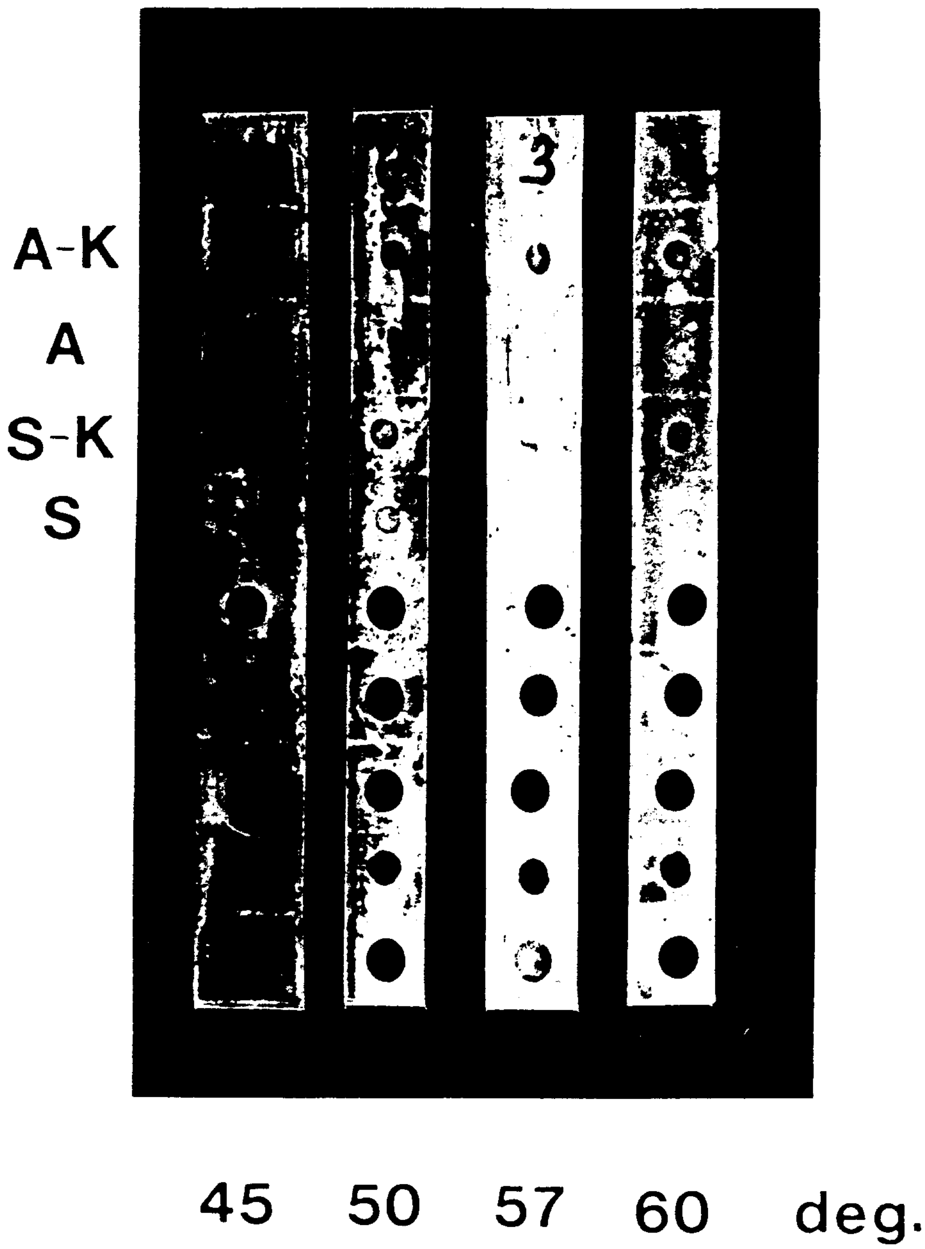


FIG. 2